# EUROPEAN PATENT APPLICATION

(11) **EP 4 394 943 A1**
(43) Date of publication of application: **03.07.2024**
(21) Application number: 22950743.9
(22) Date of filing: 15.07.2022
(51) Int. Cl.: H01M 4/505, H01M 4/525, H01M 10/0525, H01M 4/36, H01M 4/58

(54) **CONTINUOUS REACTION SYSTEM, MANGANESE IRON OXALATE PRECURSOR, LITHIUM MANGANESE IRON PHOSPHATE, PREPARATION METHOD, AND SECONDARY BATTERY**

(71) Applicant: Contemporary Amperex Technology Co., Limited, Ningde, Fujian 352100 (CN)
(72) Inventor: LIU, Shaojun, Ningde, Fujian 352100 (CN); ZHAN, Wenwei, Ningde, Fujian 352100 (CN); ZHANG, Xinxin, Ningde, Fujian 352100 (CN); OUYANG, Chuying, Ningde, Fujian 352100 (CN); LI, Qingzheng, Ningde, Fujian 352100 (CN)
(74) Representative: Kraus & Lederer PartGmbB
(86) International application number: PCT/CN2022/106086
(87) International publication number: WO 2024/011625

(57) **Abstract**

The present application provides a continuous reaction system, a ferromanganese oxalate precursor, a lithium iron manganese phosphate, a preparation method, and a secondary battery. A method for preparing a ferromanganese oxalate precursor provided in the present application is a continuous preparation method, thereby improving the production efficiency, simplifying the production process, and obtaining the ferromanganese oxalate precursor with small particle size, narrow particle size distribution, uniform element distribution, high crystallinity, regular appearance, high batch stability, and high batch consistency.

## Description

### TECHNICAL FIELD

The present application belongs to the technical field of batteries, and specifically relates to a continuous reaction system, a ferromanganese oxalate precursor, a lithium iron manganese phosphate, a preparation method, and a secondary battery.

### BACKGROUND

In recent years, secondary batteries are widely applied in energy storage power systems, such as water, fire, wind, and solar power stations, as well as many fields, such as electric tools, electric bicycles, electric motorcycles, electric vehicles, military equipment, and aerospace. With the application and promotion of the secondary batteries, their safety performance has attracted more and more attention. Because of having advantages such as high capacity, good safety performance, and abundant sources of raw materials, a lithium iron manganese phosphate has become one of the positive electrode active materials attracting greatest attention at present. A ferromanganese oxalate is one of the important raw materials for preparing the lithium iron manganese phosphate, because its performance is crucial to performance of the lithium iron manganese phosphate and the secondary batteries. However, at present, the ferromanganese oxalate is obtained by an intermittent preparation method, which has problems such as low production efficiency, complex process, uncontrollable production process, large product quality fluctuation, poor batch stability, and poor batch consistency.

### SUMMARY OF THE INVENTION

An object of the present application is to provide a continuous reaction system, a ferromanganese oxalate precursor, a lithium iron manganese phosphate, a preparation method, and a secondary battery, so as to improve the production efficiency of the ferromanganese oxalate precursor, simplify the production process of the ferromanganese oxalate precursor, and obtain the ferromanganese oxalate precursor with small particle size, narrow particle size distribution, uniform element distribution, high crystallinity, regular appearance, high batch stability, and high batch consistency.

A first aspect of the present application provides a continuous reaction system for preparing a ferromanganese oxalate precursor, including a first dissolution reactor, a second dissolution reactor, a first reactor, a second reactor, a material storage tank, and an ultrasonic reactor; where the first dissolution reactor is configured to accommodate a metal salt solution required for preparing the ferromanganese oxalate precursor, and the second dissolution reactor is configured to accommodate a precipitant solution required for preparing the ferromanganese oxalate precursor; the first reactor includes a first feed port and a first overflow port, and the first feed port of the first reactor is interconnected to a first discharge port of the first dissolution reactor and a second discharge port of the second dissolution reactor respectively through two pipelines, so that the first reactor accommodates a metal salt solution and the precipitant solution, and allows the metal salt solution and the precipitant solution to be mixed for reaction to generate a first reaction mixture; the second reactor includes a second feed port and a second overflow port, and the second feed port of the second reactor is interconnected to the first overflow port of the first reactor through a pipeline, so that the second reactor accommodates the first reaction mixture from the first reactor and allows the first reaction mixture to continue reaction to generate a second reaction mixture; the material storage tank includes a third feed port, a fourth feed port, a third discharge port, and a third overflow port, the third feed port of the material storage tank is interconnected to the second overflow port of the second reactor through a pipeline, so that the material storage tank accommodates the second reaction mixture from the second reactor and allows the second reaction mixture to continue reaction to generate a third reaction mixture, and the third discharge port and the fourth feed port of the material storage tank are circularly interconnected to the ultrasonic reactor through a circulating pipeline and a circulating pump, so that the third reaction mixture in the material storage tank is refined under ultrasonic cavitation; and when a liquid level of the third reaction mixture is higher than the third overflow port of the material storage tank, the third reaction mixture flows out through the third overflow port of the material storage tank.

In any embodiment of the present application, the continuous reaction system further includes a first metering pump and a second metering pump, two ends of the first metering pump are interconnected to the first discharge port of the first dissolution reactor and the first feed port of the first reactor respectively through pipelines to regulate a flow rate of the metal salt solution, and two ends of the second metering pump are interconnected to the second discharge port of the second dissolution reactor and the first feed port of the first reactor respectively through pipelines to regulate a flow rate of the precipitant solution.

In any embodiment of the present application, the continuous reaction system further includes a cooling water circulating pipeline arranged on an outer side of the ultrasonic reactor.

A second aspect of the present application provides a method for preparing a ferromanganese oxalate precursor through the continuous reaction system in the first aspect of the present application, at least including steps of: S1: adding a metal salt solution required for preparing the ferromanganese oxalate precursor into a first dissolution reactor, and adding a precipitant solution required for preparing the ferromanganese oxalate precursor into a second dissolution reactor; S2: transporting the metal salt solution in the first dissolution reactor and the precipitant solution in the second dissolution reactor to a first reactor respectively through different pipelines, allowing the metal salt solution and the precipitant solution to be mixed for reaction to generate a first reaction mixture, automatically transporting, when a liquid level of the first reaction mixture is higher than an overflow port of the first reactor, the first reaction mixture to a second reactor for further reaction to generate a second reaction mixture, automatically transporting, when a liquid level of the second reaction mixture is higher than an overflow port of the second reactor, the second reaction mixture to a material storage tank for further reaction to generate a third reaction mixture, pumping the third reaction mixture into an ultrasonic reactor through a circulating pipeline and a circulating pump, refining crystal particles in the third reaction mixture under ultrasonic cavitation of the ultrasonic reactor, and then re-pumping the third reaction mixture into the material storage tank, where, when a liquid level of the third reaction mixture is higher than an overflow port of the material storage tank, the third reaction mixture automatically flows out through the overflow port of the material storage tank, and during the reaction, the first dissolution reactor, the second dissolution reactor, the first reactor, the second reactor, and the material storage tank are each in a protective gas atmosphere, and each remains stirred; and S3: centrifuging, washing, and drying the third reaction mixture obtained from the overflow port of the material storage tank to obtain the ferromanganese oxalate precursor.

The method for preparing a ferromanganese oxalate precursor provided in the present application is a continuous preparation method having advantages such as high production efficiency, high energy efficiency, simple process, easy operations, and low labor costs, and is especially suitable for large-scale industrial production. The ferromanganese oxalate precursor obtained from the preparation method provided in the present application has advantages of small particle size, narrow particle size distribution, uniform element distribution, high crystallinity, regular appearance, high batch stability, and high batch consistency. Further, when the ferromanganese oxalate precursor is used as a raw material to prepare a lithium iron manganese phosphate by solid phase sintering, lithium element and various metal elements can be sufficiently mixed, so that lithium ions have a faster diffusion speed, and are easier to be intercalated into the lithium iron manganese phosphate precursor, and then the resulting lithium iron manganese phosphate has excellent electrochemical performance. The method for preparing a ferromanganese oxalate precursor provided in the present application further has good production flexibility, and can further control the crystal growth speed and regulate the size and appearance of crystal particles, thereby satisfying different production requirements to facilitate the preparation of the lithium iron manganese phosphate with different particle sizes.

In any embodiment of the present application, a complexing agent is also added into the first dissolution reactor; optionally, the complexing agent includes one or more of an aminocarboxylate, a hydroxycarboxylate, and an organic phosphonate; and more optionally, the complexing agent includes one or more of EDTMPS, sodium edetate, sodium gluconate, and sodium citrate. Therefore, ferromanganese oxalate precursor particles with high purity (for example, purity≥99.7%) and uniform distribution of metal elements can be obtained, and a molar ratio of each metal element in the resulting ferromanganese oxalate precursor particles is less different from a molar ratio of each metal element in raw materials, thereby accurately controlling metal element contents.

In any embodiment of the present application, a reaction temperature in the first reactor is lower than a reaction temperature in the second reactor, and a reaction temperature in the material storage tank is lower than the reaction temperature in the second reactor, thereby not only regulating a particle size of the resulting ferromanganese oxalate precursor, but also contributing to obtaining the ferromanganese oxalate precursor particles with narrow particle size distribution, uniform element distribution, high crystallinity, and regular appearance.

In any embodiment of the present application, the reaction temperature in the first reactor is from 20 °C to 30 °C.

In any embodiment of the present application, the reaction temperature in the second reactor is from 40 °C to 90 °C, and is optionally from 40 °C to 60 °C.

In any embodiment of the present application, the reaction temperature in the material storage tank is from 20 °C to 30 °C.

In any embodiment of the present application, a flow rate of the metal salt solution is from 0.5 L/min to 6 L/min, and is optionally from 2 L/min to 6 L/min.

In any embodiment of the present application, a flow rate of the precipitant solution is from 0.5 L/min to 6 L/min, and is optionally from 2 L/min to 6 L/min.

In any embodiment of the present application, the flow rate of the metal salt solution is equal to the flow rate of the precipitant solution, thereby contributing to improving the consistency of the resulting ferromanganese oxalate precursor particles.

In any embodiment of the present application, a residence time of the ferromanganese oxalate precursor in the first reactor during growth is from 10 min to 2 h, and is optionally from 10 min to 30 min.

In any embodiment of the present application, a residence time of the ferromanganese oxalate precursor in the second reactor during growth is from 10 min to 10 h, is optionally from 30 min to 6 h, and is more optionally from 30 min to 90 min.

In any embodiment of the present application, a residence time of the ferromanganese oxalate precursor in the material storage tank during growth is from 10 min to 10 h, is optionally from 30 min to 6 h, and is more optionally from 30 min to 90 min.

In any embodiment of the present application, a volume of the first reactor is less than or equal to a volume of the second reactor; and a ratio of the volume of the first reactor to the volume of the second reactor is optionally 1:(1-5), and is more optionally 1:3, so that the resulting ferromanganese oxalate precursor particles have higher crystallinity.

In any embodiment of the present application, the volume of the first reactor is less than or equal to a volume of the material storage tank; and a ratio of the volume of the first reactor to the volume of the material storage tank is optionally 1:(1-5), and is more optionally 1:3, so that the resulting ferromanganese oxalate precursor particles have a smaller particle size.

In any embodiment of the present application, the volume of the second reactor is equal to the volume of the material storage tank.

In any embodiment of the present application, a frequency of the ultrasonic reactor is from 15 KHz to 60 KHz, and is optionally from 30 KHz to 60 KHz, thereby contributing to the preparation of a nano-level ferromanganese oxalate precursor.

In any embodiment of the present application, the metal salt required for preparing the ferromanganese oxalate precursor includes a water-soluble ferrous salt, a water-soluble manganous salt, and an optional water-soluble divalent salt of a doping element M, where M represents a manganese-doped and iron-doped element, optionally including one or more of Co, Mg, Zn, Ca, Ti, V, Ni, or Cr.

In any embodiment of the present application, optionally, the water-soluble ferrous salt includes one or more of ferrous chloride, ferrous bromide, ferrous nitrate, ferrous sulfate, ferrous acetate, ferrous fluorosilicate, or ferrous perchlorate.

In any embodiment of the present application, optionally, the water-soluble manganous salt includes one or more of manganous chloride, manganous bromide, manganous nitrate, manganous sulfate, manganous acetate, or manganous perchlorate.

In any embodiment of the present application, optionally, the water-soluble divalent salt of the doping element M includes one or more of a chloride, a nitrate, a sulfate, or an acetate of the doping element M.

In any embodiment of the present application, the precipitant includes one or more of oxalic acid or a water-soluble oxalate, and optionally, the water-soluble oxalate includes one or more of lithium oxalate, sodium oxalate, potassium oxalate, and ammonium oxalate.

In any embodiment of the present application, a concentration of the metal salt solution is from 0.5 mol/L to 2 mol/L, and is optionally from 0.5 mol/L to 1 mol/L.

In any embodiment of the present application, a concentration of the precipitant solution is from 0.5 mol/L to 2 mol/L, and is optionally from 0.5 mol/L to 1 mol/L.

In any embodiment of the present application, a molar ratio of the metal salt to the precipitant is from 1: 1 to 1:5.

In any embodiment of the present application, a stirring speed in the first dissolution reactor is from 300 r/min to 600 r/min.

In any embodiment of the present application, a stirring speed in the second dissolution reactor is from 300 r/min to 600 r/min.

In any embodiment of the present application, a stirring speed in the first reactor is from 300 r/min to 600 r/min.

In any embodiment of the present application, a stirring speed in the second reactor is from 300 r/min to 600 r/min.

In any embodiment of the present application, a stirring speed in the material storage tank is from 300 r/min to 600 r/min.

In any embodiment of the present application, the protective gas includes nitrogen, an inert gas, or a combination thereof.

A third aspect of the present application provides a ferromanganese oxalate precursor prepared through the method in the second aspect of the present application, having a chemical formula FeₓMn_{y}M_{1-x-y}C₂O₄ •·2H₂O, 0<x<1, optionally 0.2≤x≤0.5 and 0<y<1, optionally 0.5≤y≤0.8 and 0≤1-x-y<1, and optionally 0<1-x-y≤0.05, where M represents a manganese-doped and iron-doped element, optionally including one or more of Co, Mg, Zn, Ca, Ti, V, Ni, or Cr, and the ferromanganese oxalate precursor is electroneutral.

The ferromanganese oxalate precursor provided in the present application has advantages of small particle size, narrow particle size distribution, uniform element distribution, high crystallinity, and regular appearance.

In any embodiment of the present application, volumetric particle sizes Dv90 and Dv50 of the ferromanganese oxalate precursor satisfy 1<Dv90/Dv50≤2, and optionally 1.3≤Dv90/Dv50≤1.7.

In any embodiment of the present application, the volumetric particle size Dv50 of the ferromanganese oxalate precursor is from 200 nm to 600 nm, and is optionally from 230 nm to 510 nm.

In any embodiment of the present application, the volumetric particle size Dv90 of the ferromanganese oxalate precursor is from 260 nm to 800 nm, and is optionally from 320 nm to 730 nm.

A fourth aspect of the present application provides a method for preparing a lithium iron manganese phosphate, at least including steps of: S10: sufficiently mixing the ferromanganese oxalate precursor prepared through the method in the second aspect of the present application or the ferromanganese oxalate precursor in the third aspect of the present application with a lithium source, a phosphorus source, an optional source of a doping element N, an optional source of a doping element Q, and an optional source of a doping element R at a predetermined ratio to obtain mixed raw materials, where N represents a lithium-doped element, optionally including one or more of Zn, Al, Na, K, Mg, Nb, Mo, and W, Q represents a phosphorus-doped element, optionally including one or more of B, S, Si, and N, and R represents an oxygen-doped element, optionally including one or more of S, F, Cl, and Br; and S20: sintering the mixed raw materials obtained in S10 to obtain the lithium iron manganese phosphate, where the lithium iron manganese phosphate has a chemical formula LiₐN_{b}FeₓMn_{y}M_{1-x-y}P₁₋ₘQₘO₄₋ₙRₙ, M represents a manganese-doped and iron-doped element, optionally including one or more of Co, Mg, Zn, Ca, Ti, V, Ni, or Cr, N represents a lithium-doped element, optionally including one or more of Zn, Al, Na, K, Mg, Nb, Mo, and W, Q represents a phosphorus-doped element, optionally including one or more of B, S, Si, and N, R represents an oxygen-doped element, optionally including one or more of S, F, Cl, and Br, 0.9≤a≤1.1, 0≤b≤0.1, optionally 0<b≤0.05 and 0<x<1, optionally 0.2≤x≤0.5 and 0<y<1, optionally 0.5≤y≤0.8 and 0≤1-x-y<1, optionally 0<1-x-y≤0.05 and 0≤m≤0.1, optionally 0<m≤0.05 and 0≤n≤0.1, and optionally 0<n≤0.05, and the lithium iron manganese phosphate is electroneutral.

The lithium iron manganese phosphate obtained from the above preparation method in the present application can achieve sufficiently mixing of lithium element and various metal elements, so that lithium ions have a faster diffusion speed, and are easier to be intercalated into the lithium iron manganese phosphate precursor, and then the resulting lithium iron manganese phosphate has excellent electrochemical performance.

In any embodiment of the present application, in S10, a carbon source is also added into the mixed raw materials, so that a carbon-cladded lithium iron manganese phosphate can be prepared.

A fifth aspect of the present application provides a lithium iron manganese phosphate prepared through the method in the fourth aspect of the present application, which can have excellent electrochemical performance.

A sixth aspect of the present application provides a secondary battery, comprising the lithium iron manganese phosphate prepared through the method in the fourth aspect of the present application.

### DESCRIPTION OF DRAWINGS

In order to more clearly illustrate the technical solutions in examples of the present application, the drawings to be used in the examples of the present application will be briefly introduced below. Apparently, the drawings described below are merely some embodiments of the present application. For those of ordinary skills in the art, other drawings may also be obtained based on these drawings without making creative work. In the drawings, the figures may not be drawn to the actual scale.
Fig. 1 shows a continuous reaction system for preparing a ferromanganese oxalate precursor.
Fig. 2 shows a scanning electron microscope (SEM) image of the ferromanganese oxalate precursor prepared in Example 1. A magnification in Fig. 2(a) is 20,000, and a magnification in Fig. 2(b) is 50,000.
Fig. 3 shows a scanning electron microscope (SEM) image of the ferromanganese oxalate precursor prepared in Comparative Example 3 at a magnification of 5,000.
Fig. 4 shows an X-ray diffraction (XRD) pattern of the ferromanganese oxalate precursor prepared in Example 1.

Reference numerals are as follows: 1. first dissolution reactor; 2. second dissolution reactor; 3. first reactor; 4 second reactor; 5. material storage tank; 6. ultrasonic reactor; 7. first feed port; 8. first overflow port; 9. first discharge port; 10. second discharge port; 11. second feed port; 12. second overflow port; 13. third feed port; 14. fourth feed port; 15. third discharge port; 16. third overflow port; 17. circulating pump; 18. first metering pump; 19. second metering pump; 20. cooling water circulating pipeline; 21. first stop valve; 22. second stop valve; 23. third stop valve; 24. fourth stop valve; and 25. fifth stop valve.

### DETAILED DESCRIPTION

Embodiments of a continuous reaction system, a ferromanganese oxalate precursor, a lithium iron manganese phosphate, a preparation method, and a secondary battery of the present application are specifically disclosed below with appropriate reference to the drawings. However, there will be cases where unnecessary detailed descriptions are omitted. For example, there are cases where detailed descriptions of well-known items and repeated descriptions of actually identical structures are omitted. This is to avoid unnecessary redundancy in the following descriptions and to facilitate the understanding by those skilled in the art. In addition, the drawings and subsequent descriptions are provided for those skilled in the art to fully understand the present application, and are not intended to limit the subject matter recited in the claims.

The "range" disclosed in the present application is defined in terms of lower and upper limits, and a given range is defined by selecting a lower limit and an upper limit, which define the boundaries of a particular range. A range defined in this manner may be inclusive or exclusive of end values, and may be arbitrarily combined, that is, any lower limit may be combined with any upper limit to form a range. For example, if the ranges of 60-120 and 80-110 are listed for a particular parameter, it is understood that the ranges of 60-110 and 80-120 are also contemplated. Additionally, if the minimum range values 1 and 2 are listed, and if the maximum range values 3, 4 and 5 are listed, the following ranges are all contemplated: 1-3, 1-4, 1-5, 2- 3, 2-4 and 2-5. In the present application, unless stated otherwise, the numerical range "a-b" represents an abbreviated representation of any combination of real numbers between a and b, where both a and b are real numbers. For example, the numerical range "0-5" means that all real numbers between "0-5" have been listed herein, and "0-5" is just an abbreviated representation of the combination of these numerical values. In addition, when a parameter is expressed as an integer greater than or equal to 2, it is equivalent to disclosing that the parameter is, for example, an integer of 2, 3, 4, 5, 6, 7, 8, 9, 10, 11, 12, and the like.

Unless otherwise specifically stated, all embodiments and optional embodiments of the present application may be combined with each other to form new technical solutions, and such technical solutions should be considered as being included in the disclosure of the present application.

Unless otherwise specifically stated, all technical features and optional technical features of the present application may be combined with each other to form new technical solutions, and such technical solutions should be considered as being included in the disclosure of the present application.

Unless otherwise specifically stated, all steps in the present application may be performed sequentially or may be performed randomly, and are preferably performed sequentially. For example, the method includes steps S1 and S2, which means that the method may include sequentially performed steps S1 and S2, or may include sequentially performed steps S2 and S1. For example, the reference to the method may further include step S3, which means that step S3 may be added to the method in any order, for example, the method may include steps S 1, S2, and S3, or may include steps S1, S3, and S2, or may include steps S3, S1, and S2, and so on.

Unless otherwise specifically stated, the terms "include/including" and "comprise/comprising" mentioned in the present application may be open-ended, or may be closed-ended. For example, the "including" and "comprising" may mean that other components that are not listed are further included or comprised, or only the listed components are included or comprised.

Unless otherwise specified, the term "or" is inclusive in the present application. By way of example, the phrase "A or B" means "A, B, or both A and B". More specifically, the condition "A or B" is satisfied under any one of the following conditions: A is true (or present) and B is false (or absent); A is false (or absent) and B is true (or present); or both A and B are true (or present).

Unless otherwise specifically stated, the terms such as "first," "second," and "third" in the present application are used to distinguish between different objects, and are not used to describe a specific sequence or a primary-secondary relationship.

The "plurality of" mentioned in the present application refers to two or more than two.

A first aspect of embodiments of the present application provides a continuous reaction system for preparing a ferromanganese oxalate precursor.

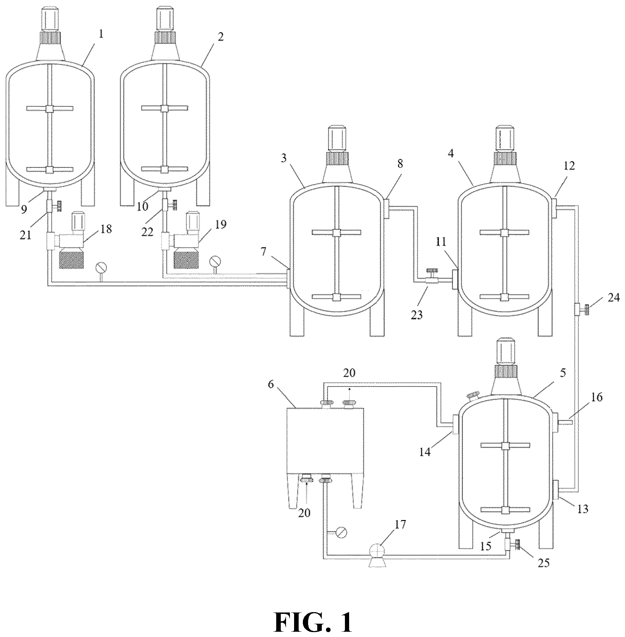

As shown in Fig. 1, the continuous reaction system includes a first dissolution reactor 1, a second dissolution reactor 2, a first reactor 3, a second reactor 4, a material storage tank 5, and an ultrasonic reactor 6; where the first dissolution reactor 1 is configured to accommodate a metal salt solution required for preparing the ferromanganese oxalate precursor, and the second dissolution reactor 2 is configured to accommodate a precipitant solution required for preparing the ferromanganese oxalate precursor; the first reactor 3 includes a first feed port 7 and a first overflow port 8, and the first feed port 7 of the first reactor is interconnected to a first discharge port 9 of the first dissolution reactor and a second discharge port 10 of the second dissolution reactor respectively through two pipelines, so that the first reactor 3 accommodates a metal salt solution and the precipitant solution, and allows the metal salt solution and the precipitant solution to be mixed for reaction to generate a first reaction mixture; the second reactor 4 includes a second feed port 11 and a second overflow port 12, and the second feed port 11 of the second reactor is interconnected to the first overflow port 8 of the first reactor through a pipeline, so that the second reactor 4 accommodates the first reaction mixture from the first reactor 3 and allows the first reaction mixture to continue reaction to generate a second reaction mixture; the material storage tank 5 includes a third feed port 13, a fourth feed port 14, a third discharge port 15, and a third overflow port 16, the third feed port 13 of the material storage tank is interconnected to the second overflow port 12 of the second reactor through a pipeline, so that the material storage tank 5 accommodates the second reaction mixture from the second reactor 4 and allows the second reaction mixture to continue reaction to generate a third reaction mixture, and the third discharge port 15 and the fourth feed port 14 of the material storage tank are circularly interconnected to the ultrasonic reactor 6 through a circulating pipeline and a circulating pump 17, so that the third reaction mixture in the material storage tank 5 is refined under ultrasonic cavitation; and when a liquid level of the third reaction mixture is higher than the third overflow port 16 of the material storage tank, the third reaction mixture flows out through the third overflow port 16 of the material storage tank.

In some examples, the continuous reaction system further includes a first metering pump 18 and a second metering pump 19, two ends of the first metering pump 18 are interconnected to the first discharge port 9 of the first dissolution reactor and the first feed port 7 of the first reactor respectively through pipelines to regulate a flow rate of the metal salt solution, and two ends of the second metering pump 19 are interconnected to the second discharge port 10 of the second dissolution reactor and the first feed port 7 of the first reactor respectively through pipelines to regulate a flow rate of the precipitant solution.

In some examples, the ultrasonic reactor 6 includes an ultrasonic reactor, an ultrasonic generator, and an ultrasonic transducer. In the present application, the working principle of the ultrasonic reactor is that a high-frequency vibration signal is sent out using the ultrasonic generator, and then converted using the ultrasonic transducer into high-frequency mechanical vibration, for transmission to the third reaction mixture uninterruptedly flowing through the ultrasonic reactor, so as to refine crystal particles (namely, ferromanganese oxalate precursor particles) in the third reaction mixture.

In some examples, the continuous reaction system further includes a cooling water circulating pipeline 20 arranged on an outer side of the ultrasonic reactor 6. When the ultrasonic reactor is used, some energy may heat the body of the ultrasonic reactor, and the cooling water circulating pipeline may be arranged to reduce the body temperature in the ultrasonic reactor, thus achieving the purpose of protecting the device.

In some examples, a first stop valve 21 is further provided on the pipeline between the first discharge port 9 of the first dissolution reactor and the first metering pump 18.

In some examples, a second stop valve 22 is further provided on the pipeline between the first discharge port 9 of the first dissolution reactor and the second metering pump 19.

In some examples, a third stop valve 23 is further provided on the pipeline between the first overflow port 8 of the first reactor and the second feed port 11 of the second reactor.

In some examples, a fourth stop valve 24 is further provided on the pipeline between the second overflow port 12 of the second reactor and the third feed port 13 of the material storage tank.

In some examples, a fifth stop valve 25 is further provided on the circulating pipeline between the third discharge port 15 of the material storage tank and the circulating pump 17.

When the ferromanganese oxalate precursor is prepared, the first stop valve 21, the second stop valve 22, the third stop valve 23, the fourth stop valve 24, and the fifth stop valve 25 can all be kept switched on, thereby ensuring continuous feeding and continuous discharging, and performing reactions in a plurality of reactors simultaneously.

In some examples, a stirring apparatus is provided in each of the first dissolution reactor 1, the second dissolution reactor 2, the first reactor 3, the second reactor 4, and the material storage tank 5.

In some examples, a heating apparatus may be further provided in the first dissolution reactor 1, the second dissolution reactor 2, the first reactor 3, the second reactor 4, and the material storage tank 5, so as to regulate the temperature in each reactor/tank as required.

In some examples, the first overflow port 8 is arranged at the top of the first reactor 3, the second overflow port 12 is arranged at the top of the second reactor 4, and the third overflow port 16 is arranged at the top of the material storage tank 5.

The second aspect of embodiments of the present application provides a method for preparing a ferromanganese oxalate precursor through the continuous reaction system in the first aspect of the embodiments of the present application, at least including steps of: S1: adding a metal salt solution required for preparing the ferromanganese oxalate precursor into a first dissolution reactor, and adding a precipitant solution required for preparing the ferromanganese oxalate precursor into a second dissolution reactor; S2: transporting the metal salt solution in the first dissolution reactor and the precipitant solution in the second dissolution reactor to a first reactor respectively through different pipelines, allowing the metal salt solution and the precipitant solution to be mixed for reaction to generate a first reaction mixture, automatically transporting, when a liquid level of the first reaction mixture is higher than an overflow port of the first reactor, the first reaction mixture to a second reactor for further reaction to generate a second reaction mixture, automatically transporting, when a liquid level of the second reaction mixture is higher than an overflow port of the second reactor, the second reaction mixture to a material storage tank for further reaction to generate a third reaction mixture, pumping the third reaction mixture into an ultrasonic reactor through a circulating pipeline and a circulating pump, refining crystal particles in the third reaction mixture under ultrasonic cavitation of the ultrasonic reactor, and then re-pumping the third reaction mixture into the material storage tank, where, when a liquid level of the third reaction mixture is higher than an overflow port of the material storage tank, the third reaction mixture automatically flows out through the overflow port of the material storage tank, and during the reaction, the first dissolution reactor, the second dissolution reactor, the first reactor, the second reactor, and the material storage tank are each in a protective gas atmosphere, and each remains stirred; and S3: centrifuging, washing, and drying the third reaction mixture obtained from the overflow port of the material storage tank to obtain the ferromanganese oxalate precursor.

Existing reaction systems for preparing a lithium iron manganese oxalate precursor mostly adopt an intermittent production process of a single reactor or a plurality of parallel-connected reactors, thereby having a defect of a low production efficiency, and when a plurality of parallel-connected reactors is used for production, it is further necessary to regularly switch between different reactors, thereby having defects of complex production process and high labor costs.

In the present application, a continuous reaction system is used to prepare the ferromanganese oxalate precursor. In the continuous reaction system of the present application, the first reactor, the second reactor, and the material storage tank are interconnected in series, thereby ensuring continuous feeding and continuous discharging, and performing reactions in the plurality of reactors simultaneously. Therefore, the method for preparing a ferromanganese oxalate precursor provided in the present application has advantages such as high production efficiency, high energy efficiency, simple process, easy operations, and low labor costs, and is especially suitable for large-scale industrial production.

The ferromanganese oxalate precursor is usually prepared by coprecipitation. During the precipitation, the growth speed of crystal nuclei is fast, and it is difficult to control the size and appearance of the resulting crystal. Therefore, the ferromanganese oxalate precursor obtained from an existing intermittent preparation method has defects of large particle size (for example, a volumetric particle size Dv50 is usually from 10 µm to 40 µm), wide particle size distribution, and non-uniform element distribution. Therefore, when the ferromanganese oxalate precursor obtained from the existing intermittent preparation method is used as a raw material to prepare a lithium iron manganese phosphate, it takes a long time to reduce its particle size by ball-milling, which is time-consuming and energy-consuming; and it is also difficult to sufficiently mix materials in a process of preparing the lithium iron manganese phosphate. In addition, the lithium iron manganese oxalate precursor obtained from the existing intermittent preparation method further has defects of large product quality fluctuation, poor batch stability, and poor batch consistency.

The method for preparing a ferromanganese oxalate precursor provided in the present application is a continuous preparation method. In the continuous preparation process, the ferromanganese oxalate precursor particles stay for a same duration in the first reactor, the second reactor, and the material storage tank during growth, and also undergo ultrasonic cavitation at a same frequency. Therefore, the particle size of the ferromanganese oxalate precursor can also be refined under a strong shear force generated by the ultrasonic cavitation. Therefore, compared with the ferromanganese oxalate precursor obtained from the existing intermittent preparation method, the ferromanganese oxalate precursor obtained from the continuous preparation method provided in the present application has advantages of small particle size, narrow particle size distribution, uniform element distribution, high crystallinity, regular appearance, high batch stability, and high batch consistency. Further, when the ferromanganese oxalate precursor is used as a raw material to prepare a lithium iron manganese phosphate by solid phase sintering, lithium element and various metal elements can be sufficiently mixed, so that lithium ions have a faster diffusion speed, and are easier to be intercalated into the lithium iron manganese phosphate precursor, and then the resulting lithium iron manganese phosphate has excellent electrochemical performance.

In the method for preparing a ferromanganese oxalate precursor provided in the present application, parameters such as the flow rate of the metal salt solution and the precipitant solution, the residence time of the ferromanganese oxalate precursor in each reactor/tank during growth, the reaction temperature in each reactor/tank, and the frequency of the ultrasonic reactor can be accurately adjusted, so that the method for preparing a ferromanganese oxalate precursor provided in the present application further has good production flexibility.

The residence time of the ferromanganese oxalate precursor during growth in each of the first reactor, the second reactor, and the material storage tank is negatively correlated with the flow rate of the metal salt solution and the precipitant solution, and is positively correlated with the volume of each reactor/tank. When the flow rate of the metal salt solution and the precipitant solution is high, the residence time of the ferromanganese oxalate precursor in each reactor/tank is short; when the flow rate of the metal salt solution and the precipitant solution is low, the residence time of the ferromanganese oxalate precursor in each reactor/tank is long; when the volume of each reactor/tank is small, the residence time of the ferromanganese oxalate precursor in each reactor/tank is short; and when the volume of each reactor/tank is large, the residence time of the ferromanganese oxalate precursor in each reactor/tank is long. Therefore, the flow rate of the metal salt solution and the precipitant solution and the volume of each reactor/tank can be adjusted to regulate the particle size and the appearance of the ferromanganese oxalate precursor.

The reaction temperature will also affect the particle size of the resulting ferromanganese oxalate precursor. Therefore, the reaction temperature in each reactor/tank can also be adjusted within different ranges to regulate the particle size and the appearance of the ferromanganese oxalate precursor.

The frequency of the ultrasonic reactor will also affect the particle size of the resulting ferromanganese oxalate precursor. Therefore, the frequency of the ultrasonic reactor may also be adjusted to regulate the particle size of the ferromanganese oxalate precursor.

Therefore, the method for preparing a ferromanganese oxalate precursor provided in the present application can further control the crystal growth speed and regulate the size and appearance of crystal particles, thereby satisfying different production requirements to facilitate the preparation of the lithium iron manganese phosphate with different particle sizes.

In some examples, a complexing agent may also be added into the first dissolution reactor.

When the ferromanganese oxalate precursor is prepared by coprecipitation, due to the difference in the precipitation speed of different metal ions, it is impossible to achieve uniform coprecipitation; and further, a molar ratio of each metal element in the resulting ferromanganese oxalate precursor particles is quite different from a molar ratio of each metal element in raw materials, thereby affecting the performance and consistency of the product. In the method for preparing a ferromanganese oxalate precursor provided in the present application, the complexing agent may also be added into the first dissolution reactor. The complexing agent can complex metal ions to achieve the purpose of controlling free metal ions, thereby improving the precipitation conversion efficiency of metal ions, reducing the difference in precipitation speed of different metal ions in the reaction mixture, and achieving uniform coprecipitation. Therefore, when the complexing agent is also added into the first dissolution reactor, the ferromanganese oxalate precursor particles with high purity (for example, purity≥99.7%) and uniform distribution of metal elements can be obtained from the method for preparing a ferromanganese oxalate precursor provided in the present application, and the molar ratio of each metal element in the resulting ferromanganese oxalate precursor particles is less different from the molar ratio of each metal element in the raw materials, thereby accurately controlling metal element contents.

Optionally, the complexing agent includes one or more of an aminocarboxylate, a hydroxycarboxylate, and an organic phosphonate. More optionally, the complexing agent includes one or more of EDTMPS, sodium edetate, sodium gluconate, and sodium citrate.

A mass concentration of the complexing agent is optionally below 10 wt%, and is more optionally from 1 wt% to 10 wt%, based on a total mass of the metal salt solution.

In some examples, a reaction temperature in the first reactor is lower than a reaction temperature in the second reactor, and a reaction temperature in the material storage tank is lower than the reaction temperature in the second reactor.

In the method for preparing a ferromanganese oxalate precursor provided in the present application, the first reactor, the second reactor, and the material storage tank are interconnected in series, and the reaction temperature in the first reactor and the material storage tank is set to be lower than the reaction temperature in the second reactor, so that different reactors in the continuous reaction system can mainly serve different functions. The reaction temperature in the first reactor is low, so that the metal salt solution and the precipitant solution are pre-mixed and rapidly pre-nucleated in the first reactor; and further, the low reaction temperature can also inhibit the aggregation and growth of the crystal nuclei in the first reaction mixture, and so that metal ions and oxalate ions can further coprecipitate to form a large number of crystal nuclei with uniform size and uniform element distribution, thereby contributing to better growth and crystallization of the crystal nuclei in the second reactor. The reaction temperature in the second reactor is high, so that when the reaction mixture flows from the first reactor into the second reactor, the high reaction temperature not only can provide enough energy to promote the growth and crystallization of the crystal nuclei, but also can improve the crystallinity of the formed crystal on the premise of avoiding the aggregation and growth of the crystal nuclei. The reaction temperature in the material storage tank is low, thereby achieving further crystallization and growth of crystals in the material storage tank, improving the crystallinity, uniformity, and consistency of the crystals; further, the reaction temperature in the material storage tank is low, thereby preventing the reaction mixture temperature from being too high, and preventing too hot ultrasonic reactor from affecting the service life of the ultrasonic transducer; and further, the reaction temperature in the material storage tank is lower than the reaction temperature in the second reactor, so that the reaction mixture, when flowing from the second reactor into the material storage tank, can function to cool the reaction mixture.

Therefore, the reaction temperature in each reactor/tank is adjusted to not only regulate the particle size of the resulting ferromanganese oxalate precursor, but also contribute to obtaining the ferromanganese oxalate precursor particles with narrow particle size distribution, uniform element distribution, high crystallinity, and regular appearance.

In some examples, optionally, the reaction temperature in the first reactor is from 20 °C to 30 °C.

In some examples, the reaction temperature in the second reactor is optionally from 40 °C to 90 °C, and is more optionally from 40 °C to 60 °C.

In some examples, optionally, the reaction temperature in the material storage tank is from 20 °C to 30 °C.

In some examples, a flow rate of the metal salt solution (or a pumping speed of the first metering pump) is from 0.5 L/min to 6 L/min, and is optionally from 2 L/min to 6 L/min.

In some examples, a flow rate of the precipitant solution (or a pumping speed of the second metering pump) is from 0.5 L/min to 6 L/min, and is optionally from 2 L/min to 6 L/min.

In some examples, the flow rate of the metal salt solution is equal to the flow rate of the precipitant solution (i.e., the pumping speed of the first metering pump is equal to the pumping speed of the second metering pump), thereby contributing to improving the consistency of the resulting ferromanganese oxalate precursor particles.

In some examples, a residence time of the ferromanganese oxalate precursor in the first reactor during growth is from 10 min to 2 h, and is optionally from 10 min to 30 min.

In some examples, a residence time of the ferromanganese oxalate precursor in the second reactor during growth is from 10 min to 10 h, is optionally from 30 min to 6 h, and is more optionally from 30 min to 90 min.

In some examples, a residence time of the ferromanganese oxalate precursor in the material storage tank during growth is from 10 min to 10 h, is optionally from 30 min to 6 h, and is more optionally from 30 min to 90 min.

In some examples, a volume of the first reactor is less than or equal to a volume of the second reactor, so that the residence time of the resulting ferromanganese oxalate precursor particles in the second reactor is long, and so that the resulting ferromanganese oxalate precursor particles have higher crystallinity. A ratio of the volume of the first reactor to the volume of the second reactor is optionally 1:(1-5), and is more optionally 1:3.

In some examples, the volume of the first reactor is less than or equal to a volume of the material storage tank, so that the residence time of the resulting ferromanganese oxalate precursor particles in the material storage tank is long, and so that the resulting ferromanganese oxalate precursor particles have a smaller particle size. A ratio of the volume of the first reactor to the volume of the material storage tank is optionally 1 :(1-5), and is more optionally 1:3.

In some examples, optionally, the volume of the second reactor is equal to the volume of the material storage tank.

In some examples, optionally, a frequency of the ultrasonic reactor is from 15 KHz to 60 KHz, and is optionally from 30 KHz to 60 KHz, thereby contributing to the preparation of a nano-level ferromanganese oxalate precursor.

In some examples, the metal salt required for preparing the ferromanganese oxalate precursor includes a water-soluble ferrous salt, a water-soluble manganous salt, and an optional water-soluble divalent salt of a doping element M, where M represents a manganese-doped and iron-doped element, optionally including one or more of Co, Mg, Zn, Ca, Ti, V, Ni, or Cr.

The water-soluble ferrous salt may be various existing water-soluble compounds comprising ferrous ions, and optionally, the water-soluble ferrous salt includes one or more of ferrous chloride, ferrous bromide, ferrous nitrate, ferrous sulfate, ferrous acetate, ferrous fluorosilicate, or ferrous perchlorate.

The water-soluble manganous salt may be various existing water-soluble compounds comprising ferrous ions, and optionally, the water-soluble manganous salt includes one or more of manganous chloride, manganous bromide, manganous nitrate, manganous sulfate, manganous acetate, or manganous perchlorate.

The water-soluble divalent salt of the doping element M may be various existing water-soluble compounds comprising divalent M ions, and optionally, the water-soluble divalent salt of the doping element M includes one or more of a chloride, a nitrate, a sulfate, or an acetate of the doping element M.

In some examples, optionally, the precipitant includes one or more of oxalic acid or a water-soluble oxalate. Optionally, the water-soluble oxalate includes one or more of lithium oxalate, sodium oxalate, potassium oxalate, and ammonium oxalate.

In some examples, the metal salt solution is an aqueous metal salt solution obtained by, for example, sufficiently mixing the metal salt required for preparing the ferromanganese oxalate precursor with deionized water.

In some examples, the precipitant solution is an aqueous precipitant solution obtained by, for example, sufficiently mixing the precipitant with deionized water.

In some examples, a concentration of the metal salt solution is optionally from 0.5 mol/L to 2 mol/L, and is more optionally from 0.5 mol/L to 1 mol/L.

In some examples, a concentration of the precipitant solution is optionally from 0.5 mol/L to 2 mol/L, and is more optionally from 0.5 mol/L to 1 mol/L.

In some examples, optionally, a molar ratio of the metal salt to the precipitant is from 1:1 to 1:5.

During the reaction, the first dissolution reactor, the second dissolution reactor, the first reactor, the second reactor, and the material storage tank each remain stirred. In some examples, optionally, a stirring speed in the first dissolution reactor is from 300 r/min to 600 r/min. In some examples, optionally, a stirring speed in the second dissolution reactor is from 300 r/min to 600 r/min. In some examples, optionally, a stirring speed in the first reactor is from 300 r/min to 600 r/min. In some examples, optionally, a stirring speed in the second reactor is from 300 r/min to 600 r/min. In some examples, optionally, a stirring speed in the material storage tank is from 300 r/min to 600 r/min.

During the reaction, the first dissolution reactor, the second dissolution reactor, the first reactor, the second reactor, and the material storage tank are each insufflated with a protective gas. In some examples, the protective gas includes nitrogen, an inert gas, or a combination thereof. Optionally, the inert gas includes helium, argon, or a combination thereof.

In the method for preparing a ferromanganese oxalate precursor provided in the present application, unless otherwise specifically stated, all raw materials are directly commercially available.

A third aspect of embodiments of the present application provides a ferromanganese oxalate precursor obtained from the method in the second aspect of the embodiments of the present application, having a chemical formula FeₓMn_{y}M_{1-x-y}C₂O₄·2H₂O, 0<x<1, 0<y<1, and 0≤1-x-y<1, where M represents a manganese-doped and iron-doped element, optionally including one or more of Co, Mg, Zn, Ca, Ti, V, Ni, or Cr, and the ferromanganese oxalate precursor is electroneutral.

The ferromanganese oxalate precursor provided in the present application is obtained from the method in the second aspect of the embodiments of the present application, and therefore has advantages of small particle size, narrow particle size distribution, uniform element distribution, high crystallinity, and regular appearance.

In some examples, volumetric particle sizes Dv90 and Dv50 of the ferromanganese oxalate precursor satisfy 1<Dv90/Dv50≤2, and optionally 1.3≤Dv90/Dv50≤1.7.

In some examples, the volumetric particle size Dv50 of the ferromanganese oxalate precursor is from 200 nm to 600 nm, and is optionally from 230 nm to 510 nm.

In some examples, the volumetric particle size Dv90 of the ferromanganese oxalate precursor is from 260 nm to 800 nm, and is optionally from 320 nm to 730 nm.

In some examples, optionally, 0.2≤x≤0.5.

In some examples, optionally, 0.5≤y≤0.8.

In some examples, 1-x-y=0; and in some other examples, 0<1-x-y≤0.05.

The fourth aspect of embodiments of the present application provides a method for preparing a lithium iron manganese phosphate, at least including steps of: S10: sufficiently mixing the ferromanganese oxalate precursor prepared through the method in the second aspect of the embodiments of the present application or the ferromanganese oxalate precursor in the third aspect of the embodiments of the present application with a lithium source, a phosphorus source, an optional source of a doping element N, an optional source of a doping element Q, and an optional source of a doping element R at a predetermined ratio to obtain mixed raw materials, where N represents a lithium-doped element, optionally including one or more of Zn, Al, Na, K, Mg, Nb, Mo, and W, Q represents a phosphorus-doped element, optionally including one or more of B, S, Si, and N, and R represents an oxygen-doped element, optionally including one or more of S, F, Cl, and Br; and S20: sintering the mixed raw materials obtained in S10 to obtain the lithium iron manganese phosphate, where the lithium iron manganese phosphate has a chemical formula LiₐN_{b}FeₓMn_{y}M_{1-x-y}P₁₋ₘQₘO₄₋ₙRₙ, M represents a manganese-doped and iron-doped element, optionally including one or more of Co, Mg, Zn, Ca, Ti, V, Ni, or Cr, N represents a lithium-doped element, optionally including one or more of Zn, Al, Na, K, Mg, Nb, Mo, and W, Q represents a phosphorus-doped element, optionally including one or more of B, S, Si, and N, R represents an oxygen-doped element, optionally including one or more of S, F, Cl, and Br, 0.9≤a≤1.1, 0≤b≤0.1, optionally 0<b≤0.05 and 0<x<1, optionally 0.2≤x≤0.5 and 0<y<1, optionally 0.5≤y≤0.8 and 0≤1-x-y<1, optionally 0<1-x-y≤0.05 and 0≤m≤0.1, optionally 0<m≤0.05 and 0≤n≤0.1, and optionally 0<n≤0.05, and the lithium iron manganese phosphate is electroneutral.

The lithium iron manganese phosphate obtained from the above preparation method in the present application can achieve sufficiently mixing of lithium element and various metal elements, so that lithium ions have a faster diffusion speed, and are easier to be intercalated into the lithium iron manganese phosphate precursor, and then the resulting lithium iron manganese phosphate has excellent electrochemical performance.

In some examples, the lithium source may be a lithium-containing compound usable for preparing a positive electrode active material lithium iron manganese phosphate known in the art, for example, the lithium source includes one or more of Li₂CO₃, LiOH, Li₃PO₄, and LiH₂PO₄.

In some examples, the phosphorus source may be a phosphorus-containing compound usable for preparing a positive electrode active material lithium iron manganese phosphate known in the art, for example, the phosphorus source includes one or more of (NH₄)₂HPO₄, NH₄H₂PO₄, (NH₄)₃PO₄, and H₃PO₄.

In some examples, the source of the doping element N includes one or more of a chloride, a nitrate, a sulfate, and an acetate of the doping element N.

In some examples, the source of the doping element Q includes one or more of a sulfate, a borate, a nitrate, or a silicate of the doping element Q.

In some examples, the source of the doping element R includes one or more of an elementary substance and an ammonium salt of the doping element R.

The source of each of the above doping elements is selected, thereby improving uniform distribution of each of the above doping elements, and improving the electrochemical performance of the lithium iron manganese phosphate.

In some examples, in S 10, a carbon source may also be added into the mixed raw materials, so that a carbon-cladded lithium iron manganese phosphate can be prepared.

In some examples, the carbon source includes one or more an organic carbon source or an inorganic carbon source, and optionally includes a combination of one or more of glucose, sucrose, starch, fructose, polyvinyl alcohol, polyethylene glycol, and citric acid.

In the above preparation method, an addition amount of the source of each of the doping elements N, Q, and R depends on a target doping amount; and an addition amount of the lithium source and the phosphorus source satisfies a stoichiometric ratio of the lithium iron manganese phosphate. In some examples, the addition amount of the lithium source may be slightly excessive, for example, may be from 100% to 110% of a theoretical mass of the lithium source. The theoretical mass of the lithium source refers to a mass of the lithium source computed according to the stoichiometric ratio of the lithium iron manganese phosphate.

In the above preparation method, unless otherwise specifically stated, all raw materials are directly commercially available.

The fifth aspect of embodiments of the present application provides a lithium iron manganese phosphate prepared through the method in the fourth aspect of the embodiments of the present application.

The lithium iron manganese phosphate has a chemical formula LiₐN_{b}FeₓMn_{y}M_{1-x-y}P₁₋ₘQₘO₄₋ₙRₙ, M represents a manganese-doped and iron-doped element, optionally including one or more of Co, Mg, Zn, Ca, Ti, V, Ni, or Cr, N represents a lithium-doped element, optionally including one or more of Zn, Al, Na, K, Mg, Nb, Mo, and W, Q represents a phosphorus-doped element, optionally including one or more of B, S, Si, and N, R represents an oxygen-doped element, optionally including one or more of S, F, Cl, and Br, 0.9≤a≤1.1, 0≤b≤0.1, optionally 0<b≤0.05 and 0<x<1, optionally 0.2≤x≤0.5 and 0<y<1, optionally 0.5≤y≤0.8 and 0≤1-x-y<1, optionally 0<1-x-y≤0.05 and 0≤m≤0.1, optionally 0<m≤0.05 and 0≤n≤0.1, and optionally 0<n≤0.05, and the lithium iron manganese phosphate is electroneutral.

In some examples, the surface of the lithium iron manganese phosphate is also carbon-cladded, thereby improving conductivity of the lithium iron manganese phosphate.

The sixth aspect of embodiments of the present application provides a secondary battery, comprising the lithium iron manganese phosphate prepared through the method in the fourth aspect of the embodiments of the present application. The lithium iron manganese phosphate can be used in the secondary battery as a positive electrode active material, and also contributes to improving the electrochemical performance of the secondary battery.

### Examples

The following examples describe the disclosure of the present application in more detail and are provided for illustrative purposes only, as various modifications and alterations within the scope of the disclosure of the present application will be apparent to those skilled in the art. Unless otherwise stated, all parts, percentages, and ratios reported in the following examples are on a mass basis; all reagents used in the examples are commercially available or can be synthesized according to conventional methods, and can be directly used without further treatment; and the instruments used in the examples are commercially available.

### Example 1

A ferromanganese oxalate precursor was prepared using a continuous reaction system shown in Fig. 1.

A water-soluble manganous salt manganous chloride, a water-soluble ferrous salt ferrous chloride, a complexing agent EDTMPS, and deionized water were added into a first dissolution reactor to prepare a metal salt solution at a concentration of 1 mol/L. In the metal salt solution thus prepared, a molar ratio of manganous ions to ferrous ions was 7:3, and a mass concentration of the complexing agent was 5 wt%. A precipitant ammonium oxalate and deionized water were added into a second dissolution reactor to prepare a precipitant solution at a concentration of 1 mol/L.

As shown in Fig. 1, nitrogen is insufflated into each of the first dissolution reactor, the second dissolution reactor, a first reactor, a second reactor, and a material storage tank, and a stirring speed in each of them was 400 r/min during reaction. A reaction temperature in the first reactor and a reaction temperature in the material storage tank were each controlled at room temperature (25 °C), a reaction temperature in the second reactor was controlled at 40 °C, and a frequency of the ultrasonic reactor was 60 KHz.

The metal salt solution and the precipitant solution were uninterruptedly transported to the first reactor from the first dissolution reactor and the second dissolution reactor respectively through two inlet pipelines, and were allowed to be mixed for reaction to generate a first reaction mixture, where a flow rate of the metal salt solution and a flow rate of the precipitant solution were each 6 L/min.

After reaction for 10 min, a liquid level of the first reaction mixture was higher than a first overflow port, and then the first reaction mixture automatically flowed out through the first overflow port to the second reactor for further reaction to generate a second reaction mixture.

After the reaction was continued for an additional 30 min, a liquid level of the second reaction mixture was higher than a second overflow port, and then the second reaction mixture automatically flowed out through the second overflow port to the material storage tank for further reaction to generate a third reaction mixture.

The third reaction mixture in the material storage tank was uninterruptedly pumped into the ultrasonic reactor through a circulating pipeline and a circulating pump. Ferromanganese oxalate precursor particles in the third reaction mixture were refined under ultrasonic cavitation of the ultrasonic reactor, and then re-pumped into the material storage tank.

After the reaction was continued for an additional 30 min, a liquid level of the third reaction mixture was higher than a third overflow port, and then the third reaction mixture automatically flowed out through the third overflow port.

The resulting third reaction mixture was centrifuged and washed through a centrifuge many times, and then transferred into a dryer for drying at 120 °C for 6 h to obtain the ferromanganese oxalate precursor.

### Example 2

A ferromanganese oxalate precursor was prepared using a continuous reaction system shown in Fig. 1.

A water-soluble manganous salt manganous nitrate, a water-soluble ferrous salt ferrous nitrate, a complexing agent sodium edetate, and deionized water were added into a first dissolution reactor to prepare a metal salt solution at a concentration of 1 mol/L. In the metal salt solution thus prepared, a molar ratio of manganous ions to ferrous ions was 7:3, and a mass concentration of the complexing agent was 3 wt%. A precipitant oxalic acid and deionized water were added into a second dissolution reactor to prepare a precipitant solution at a concentration of 1 mol/L.

As shown in Fig. 1, nitrogen is insufflated into each of the first dissolution reactor, the second dissolution reactor, a first reactor, a second reactor, and a material storage tank, and a stirring speed in each of them was 600 r/min during reaction. A reaction temperature in the first reactor and a reaction temperature in the material storage tank were each controlled at room temperature (25 °C), a reaction temperature in the second reactor was controlled at 60 °C, and a frequency of the ultrasonic reactor was 30 KHz.

The metal salt solution and the precipitant solution were uninterruptedly transported to the first reactor from the first dissolution reactor and the second dissolution reactor respectively through two inlet pipelines, and were allowed to be mixed for reaction to generate a first reaction mixture, where a flow rate of the metal salt solution and a flow rate of the precipitant solution were each 2 L/min.

After reaction for 30 min, a liquid level of the first reaction mixture was higher than a first overflow port, and then the first reaction mixture automatically flowed out through the first overflow port to the second reactor for further reaction to generate a second reaction mixture.

After the reaction was continued for an additional 90 min, a liquid level of the second reaction mixture was higher than a second overflow port, and then the second reaction mixture automatically flowed out through the second overflow port to the material storage tank for further reaction to generate a third reaction mixture.

The third reaction mixture in the material storage tank was uninterruptedly pumped into the ultrasonic reactor through a circulating pipeline and a circulating pump. Ferromanganese oxalate precursor particles in the third reaction mixture were refined under ultrasonic cavitation of the ultrasonic reactor, and then re-pumped into the material storage tank.

After the reaction was continued for an additional 90 min, a liquid level of the third reaction mixture was higher than a third overflow port, and then the third reaction mixture automatically flowed out through the third overflow port.

The resulting third reaction mixture was centrifuged and washed through a centrifuge many times, and then transferred into a dryer for drying at 120 °C for 6 h to obtain the ferromanganese oxalate precursor.

### Example 3

A ferromanganese oxalate precursor was prepared using a continuous reaction system shown in Fig. 1.

A water-soluble manganous salt manganous sulfate, a water-soluble ferrous salt ferrous sulfate, a complexing agent sodium gluconate, and deionized water were added into a first dissolution reactor to prepare a metal salt solution at a concentration of 0.5 mol/L. In the metal salt solution thus prepared, a molar ratio of manganous ions to ferrous ions was 6:4, and a mass concentration of the complexing agent was 10 wt%. A precipitant oxalic acid and deionized water were added into a second dissolution reactor to prepare a precipitant solution at a concentration of 0.5 mol/L.

As shown in Fig. 1, nitrogen is insufflated into each of the first dissolution reactor, the second dissolution reactor, a first reactor, a second reactor, and a material storage tank, and a stirring speed in each of them was 300 r/min during reaction. A reaction temperature in the first reactor and a reaction temperature in the material storage tank were each controlled at room temperature (25 °C), a reaction temperature in the second reactor was controlled at 50 °C, and a frequency of the ultrasonic reactor was 50 KHz.

The metal salt solution and the precipitant solution were uninterruptedly transported to the first reactor from the first dissolution reactor and the second dissolution reactor respectively through two inlet pipelines, and were allowed to be mixed for reaction to generate a first reaction mixture, where a flow rate of the metal salt solution and a flow rate of the precipitant solution were each 3 L/min.

After reaction for 20 min, a liquid level of the first reaction mixture was higher than a first overflow port, and then the first reaction mixture automatically flowed out through the first overflow port to the second reactor for further reaction to generate a second reaction mixture.

After the reaction was continued for an additional 60 min, a liquid level of the second reaction mixture was higher than a second overflow port, and then the second reaction mixture automatically flowed out through the second overflow port to the material storage tank for further reaction to generate a third reaction mixture.

The third reaction mixture in the material storage tank was uninterruptedly pumped into the ultrasonic reactor through a circulating pipeline and a circulating pump. Ferromanganese oxalate precursor particles in the third reaction mixture were refined under ultrasonic cavitation of the ultrasonic reactor, and then re-pumped into the material storage tank.

After the reaction was continued for an additional 60 min, a liquid level of the third reaction mixture was higher than a third overflow port, and then the third reaction mixture automatically flowed out through the third overflow port.

The resulting third reaction mixture was centrifuged and washed through a centrifuge many times, and then transferred into a dryer for drying at 120 °C for 6 h to obtain the ferromanganese oxalate precursor.

### Example 4

A ferromanganese oxalate precursor was prepared using a continuous reaction system shown in Fig. 1.

A water-soluble manganous salt manganous acetate, a water-soluble ferrous salt ferrous acetate, a water-soluble cobaltous salt cobaltous acetate, a complexing agent sodium citrate, and deionized water were added into a first dissolution reactor to prepare a metal salt solution at a concentration of 1 mol/L. In the metal salt solution thus prepared, a molar ratio of manganous ions, ferrous ions, and cobaltous ions was 6.9:3:0.1, and a mass concentration of the complexing agent was 5 wt%. Precipitants ammonium oxalate and oxalic acid and deionized water were added into a second dissolution reactor to prepare a precipitant solution at a concentration of 1 mol/L, where a molar ratio of ammonium oxalate to oxalic acid was 1:1.

As shown in Fig. 1, nitrogen is insufflated into each of the first dissolution reactor, the second dissolution reactor, a first reactor, a second reactor, and a material storage tank, and a stirring speed in each of them was 400 r/min during reaction. A reaction temperature in the first reactor and a reaction temperature in the material storage tank were each controlled at room temperature (25 °C), a reaction temperature in the second reactor was controlled at 60 °C, and a frequency of the ultrasonic reactor was 40 KHz.

The metal salt solution and the precipitant solution were uninterruptedly transported to the first reactor from the first dissolution reactor and the second dissolution reactor respectively through two inlet pipelines, and were allowed to be mixed for reaction to generate a first reaction mixture, where a flow rate of the metal salt solution and a flow rate of the precipitant solution were each 4 L/min.

After reaction for 15 min, a liquid level of the first reaction mixture was higher than a first overflow port, and then the first reaction mixture automatically flowed out through the first overflow port to the second reactor for further reaction to generate a second reaction mixture.

After the reaction was continued for an additional 45 min, a liquid level of the second reaction mixture was higher than a second overflow port, and then the second reaction mixture automatically flowed out through the second overflow port to the material storage tank for further reaction to generate a third reaction mixture.

The third reaction mixture in the material storage tank was uninterruptedly pumped into the ultrasonic reactor through a circulating pipeline and a circulating pump. Ferromanganese oxalate precursor particles in the third reaction mixture were refined under ultrasonic cavitation of the ultrasonic reactor, and then re-pumped into the material storage tank.

After the reaction was continued for an additional 45 min, a liquid level of the third reaction mixture was higher than a third overflow port, and then the third reaction mixture automatically flowed out through the third overflow port.

The resulting third reaction mixture was centrifuged and washed through a centrifuge many times, and then transferred into a dryer for drying at 120 °C for 6 h to obtain the ferromanganese oxalate precursor.

### Example 5

The method for preparing a ferromanganese oxalate precursor was same as that in Example 1, except that no complexing agent was added.

### Comparative Example 1

A water-soluble manganous salt manganous chloride, a water-soluble ferrous salt ferrous chloride, and deionized water were mixed to prepare a metal salt solution at a concentration of 1 mol/L. In the metal salt solution thus prepared, a molar ratio of manganous ions to ferrous ions was 7:3. A precipitant oxalic acid and deionized water were mixed to prepare a precipitant solution at a concentration of 1 mol/L.

Both the metal salt solution and the precipitant solution were injected into a reactor, rapid stirring was started at a stirring speed of 400 r/min, a reaction temperature was controlled at 60 °C, and a reaction duration was 40 min. After the reaction was complete, the mixture was naturally cooled to room temperature to obtain a slurry. The resulting slurry was discharged through a discharge port at the bottom of the reactor, centrifuged and washed through a centrifuge many times, and then transferred into a dryer for drying at 120 °C for 6 h to obtain the ferromanganese oxalate precursor.

### Comparative Example 2

A water-soluble manganous salt manganous chloride, a water-soluble ferrous salt ferrous chloride, a complexing agent EDTMPS, and deionized water were mixed to prepare a metal salt solution at a concentration of 1 mol/L. In the metal salt solution thus prepared, a molar ratio of manganous ions to ferrous ions was 7:3, and a mass concentration of the complexing agent was 5 wt%. A precipitant oxalic acid and deionized water were mixed to prepare a precipitant solution at a concentration of 1 mol/L.

Both the metal salt solution and the precipitant solution were injected into a reactor, rapid stirring was started at a stirring speed of 400 r/min, a reaction temperature was controlled at 60 °C, and a reaction duration was 40 min. After the reaction was complete, the mixture was naturally cooled to room temperature to obtain a slurry. The resulting slurry was discharged through a discharge port at the bottom of the reactor, centrifuged and washed through a centrifuge many times, and then transferred into a dryer for drying at 120 °C for 6 h to obtain the ferromanganese oxalate precursor.

### Comparative Example 3

A water-soluble manganous salt manganous chloride, a water-soluble ferrous salt ferrous chloride, and deionized water were mixed to prepare a metal salt solution at a concentration of 1 mol/L. In the metal salt solution thus prepared, a molar ratio of manganous ions to ferrous ions was 7:3. A precipitant oxalic acid and deionized water were mixed to prepare a precipitant solution at a concentration of 1 mol/L.

Both the metal salt solution and the precipitant solution were injected into a reactor, rapid stirring was started at a stirring speed of 400 r/min, a reaction temperature was controlled at 60 °C, and a reaction duration was 40 min. After the reaction was complete, the mixture was naturally cooled to room temperature to obtain a slurry. The resulting slurry was discharged through a discharge port at the bottom of the reactor, transferred into an ultrasonic reactor for refinement, kept for reaction for 30 min, then centrifuged and washed through a centrifuge many times, and then transferred into a dryer for drying at 120 °C for 6 h to obtain the ferromanganese oxalate precursor.

### Test

### (1) Testing of particle size

A volumetric particle size of the ferromanganese oxalate precursor prepared above was tested using Malvern Master Size 3000 laser particle size analyzer. Dv50 and Dv90 refer to corresponding particle sizes when a cumulative volume distribution percentage of a material reaches 50% and 90%. GB/T 19077-2016 may be referred to for the test.

### (2) Testing of Mn/Fe molar ratio

The contents of manganese and iron elements in the ferromanganese oxalate precursor prepared above were tested by ICP-OES using Plasma 3000 inductively coupled plasma emission spectrometer, and their molar ratio was computed.

**Table 1**

| Serial No. | Dv50 | Dv90 | Dv90/Dv50 | Mn/Fe molar ratio | |
|---|---|---|---|---|---|
| | | | | Added value | Test value |
| Example 1 | 230 nm | 320 nm | 1.39 | 7:3 | 2.336:1 |
| Example 2 | 510 nm | 730 nm | 1.43 | 7:3 | 2.324:1 |
| Example 3 | 320 nm | 530 nm | 1.66 | 6:4 | 1.496:1 |
| Example 4 | 350 nm | 570 nm | 1.63 | 6.9:3 | 2.297:1 |
| Example 5 | 235 nm | 340 nm | 1.45 | 7:3 | 2.174:1 |
| Comparative Example 1 | 40 µm | 85 µm | 2.13 | 7:3 | 1.947:1 |
| Comparative Example 2 | 38 µm | 80 µm | 2.11 | 7:3 | 2.265:1 |
| Comparative Example 3 | 3.5 µm | 9.5 µm | 2.71 | 7:3 | 1.945:1 |

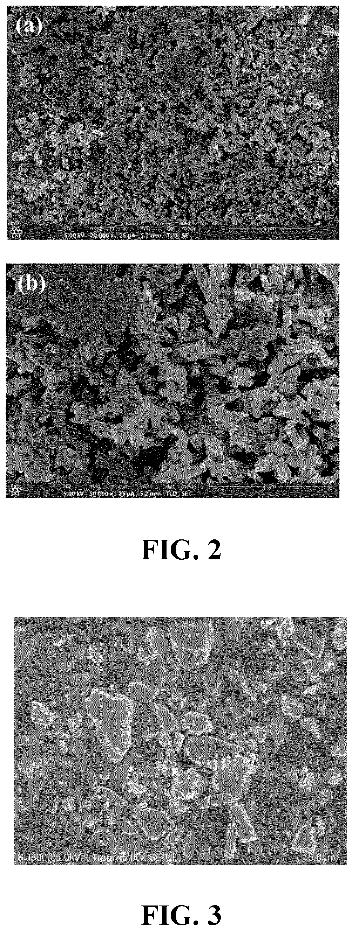

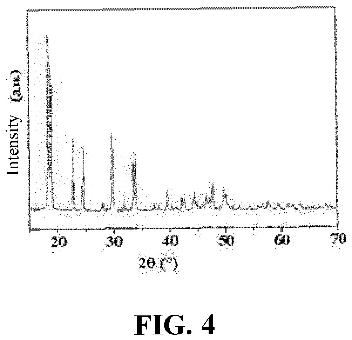

Fig. 2 shows a scanning electron microscope (SEM) image of the ferromanganese oxalate precursor prepared in Example 1. A magnification in Fig. 2(a) is 20,000, and a magnification in Fig. 2(b) is 50,000. Fig. 3 shows a scanning electron microscope (SEM) image of the ferromanganese oxalate precursor prepared in Comparative Example 3 at a magnification of 5,000. Fig. 4 shows an X-ray diffraction (XRD) pattern of the ferromanganese oxalate precursor prepared in Example 1.

As can be seen from the test result in Table 1 and Fig. 2, the ferromanganese oxalate precursor prepared through the continuous preparation method provided in the present application has advantages of small particle size, narrow particle size distribution, uniform element distribution, and regular appearance.

As can be seen from the test result in Table 1 and Fig. 3, the ferromanganese oxalate precursor prepared through the existing intermittent preparation method has large particle size, wide particle size distribution, and non-uniform element distribution.

As can be further seen from Fig. 4, the ferromanganese oxalate precursor prepared through the continuous preparation method provided in the present application further has advantages of high purity and high crystallinity.

As can be seen from the test results of Example 1 and Comparative Example 2, Example 5 and Comparative Example 1, the Mn/Fe molar ratio in the ferromanganese oxalate precursor particles prepared through the continuous preparation method provided in the present application is less different from the Mn/Fe molar ratio in the metal salt solution, thereby accurately controlling the contents of manganese element and iron element.

As can be further seen from the test results of Example 1 and Comparative Example 2, Example 5 and Comparative Example 1, when a complexing agent was added into the first dissolution reactor, it contributed to further accurately control the contents of manganese element and iron element in the resulting ferromanganese oxalate precursor particles.

It should be noted that the present application is not limited to the above embodiments. The above embodiments are merely exemplary, and embodiments having substantially same technical concept and same effects within the scope of the technical solutions of the present application are all encompassed within the technical scope of the present application. In addition, without departing from the scope of the subject matter of the present application, various modifications that can be conceived by those skilled in the art are applied to the embodiments, and other modes constructed by combining some of the constituent elements of the embodiments are also included in the scope of the present application.

## Claims

1. A continuous reaction system for preparing a ferromanganese oxalate precursor, wherein,
the continuous reaction system comprises a first dissolution reactor, a second dissolution reactor, a first reactor, a second reactor, a material storage tank, and an ultrasonic reactor;
the first dissolution reactor is configured to accommodate a metal salt solution required for preparing the ferromanganese oxalate precursor, and the second dissolution reactor is configured to accommodate a precipitant solution required for preparing the ferromanganese oxalate precursor;
the first reactor includes a first feed port and a first overflow port, and the first feed port of the first reactor is interconnected to a first discharge port of the first dissolution reactor and a second discharge port of the second dissolution reactor respectively through two pipelines, so that the first reactor accommodates a metal salt solution and the precipitant solution, and allows the metal salt solution and the precipitant solution to be mixed for reaction to generate a first reaction mixture;
the second reactor includes a second feed port and a second overflow port, and the second feed port of the second reactor is interconnected to the first overflow port of the first reactor through a pipeline, so that the second reactor accommodates the first reaction mixture from the first reactor and allows the first reaction mixture to continue reaction to generate a second reaction mixture;
the material storage tank comprises a third feed port, a fourth feed port, a third discharge port, and a third overflow port, the third feed port of the material storage tank is interconnected to the second overflow port of the second reactor through a pipeline, so that the material storage tank accommodates the second reaction mixture from the second reactor and allows the second reaction mixture to continue reaction to generate a third reaction mixture, and the third discharge port and the fourth feed port of the material storage tank are circularly interconnected to the ultrasonic reactor through a circulating pipeline and a circulating pump, so that the third reaction mixture in the material storage tank is refined under ultrasonic cavitation; and
when a liquid level of the third reaction mixture is higher than the third overflow port of the material storage tank, the third reaction mixture flows out through the third overflow port of the material storage tank.

2. The continuous reaction system according to claim 1, wherein the continuous reaction system further comprises a first metering pump and a second metering pump, two ends of the first metering pump are interconnected to the first discharge port of the first dissolution reactor and the first feed port of the first reactor respectively through pipelines to regulate a flow rate of the metal salt solution, and two ends of the second metering pump are interconnected to the second discharge port of the second dissolution reactor and the first feed port of the first reactor respectively through pipelines to regulate a flow rate of the precipitant solution.

3. The continuous reaction system according to claim 1 or 2, wherein the continuous reaction system further comprises a cooling water circulating pipeline arranged on an outer side of the ultrasonic reactor.

4. A method for preparing a ferromanganese oxalate precursor through the continuous reaction system according to any one of claims 1-3, at least comprising steps of:
S1: adding a metal salt solution required for preparing the ferromanganese oxalate precursor into a first dissolution reactor, and adding a precipitant solution required for preparing the ferromanganese oxalate precursor into a second dissolution reactor;
S2: transporting the metal salt solution in the first dissolution reactor and the precipitant solution in the second dissolution reactor to a first reactor respectively through different pipelines, allowing the metal salt solution and the precipitant solution to be mixed for reaction to generate a first reaction mixture, automatically transporting, when a liquid level of the first reaction mixture is higher than an overflow port of the first reactor, the first reaction mixture to a second reactor for further reaction to generate a second reaction mixture, automatically transporting, when a liquid level of the second reaction mixture is higher than an overflow port of the second reactor, the second reaction mixture to a material storage tank for further reaction to generate a third reaction mixture, pumping the third reaction mixture into an ultrasonic reactor through a circulating pipeline and a circulating pump, refining crystal particles in the third reaction mixture under ultrasonic cavitation of the ultrasonic reactor, and then re-pumping the third reaction mixture into the material storage tank, where, when a liquid level of the third reaction mixture is higher than an overflow port of the material storage tank, the third reaction mixture automatically flows out through the overflow port of the material storage tank, and during the reaction, the first dissolution reactor, the second dissolution reactor, the first reactor, the second reactor, and the material storage tank are each in a protective gas atmosphere, and each remains stirred; and
S3: centrifuging, washing, and drying the third reaction mixture obtained from the overflow port of the material storage tank to obtain the ferromanganese oxalate precursor.

5. The method according to claim 4, wherein a complexing agent is also added into the first dissolution reactor, optionally, the complexing agent includes one or more of an aminocarboxylate, a hydroxycarboxylate, and an organic phosphonate, and more optionally, the complexing agent includes one or more of EDTMPS, sodium edetate, sodium gluconate, and sodium citrate.

6. The method according to claim 4 or 5, wherein a reaction temperature in the first reactor is lower than a reaction temperature in the second reactor, and a reaction temperature in the material storage tank is lower than the reaction temperature in the second reactor.

7. The method according to claim 6, wherein,
the reaction temperature in the first reactor is from 20 °C to 30 °C; and/or,
the reaction temperature in the second reactor is from 40 °C to 90 °C, and is optionally from 40 °C to 60 °C; and/or,
the reaction temperature in the material storage tank is from 20 °C to 30 °C.

8. The method according to any one of claims 4-7, wherein,
a flow rate of the metal salt solution is from 0.5 L/min to 6 L/min, and is optionally from 2 L/min to 6 L/min; and/or,
a flow rate of the precipitant solution is from 0.5 L/min to 6 L/min, and is optionally from 2 L/min to 6 L/min; and/or,
the flow rate of the metal salt solution is equal to the flow rate of the precipitant solution.

9. The method according to any one of claims 4-8, wherein,
a residence time of the ferromanganese oxalate precursor in the first reactor during growth is from 10 min to 2 h, and is optionally from 10 min to 30 min; and/or,
a residence time of the ferromanganese oxalate precursor in the second reactor during growth is from 10 min to 10 h, is optionally from 30 min to 6 h, and is more optionally from 30 min to 90 min; and/or,
a residence time of the ferromanganese oxalate precursor in the material storage tank during growth is from 10 min to 10 h, is optionally from 30 min to 6 h, and is more optionally from 30 min to 90 min.

10. The method according to any one of claims 4-9, wherein,
a volume of the first reactor is less than or equal to a volume of the second reactor; and a ratio of the volume of the first reactor to the volume of the second reactor is optionally 1:(1-5), and is more optionally 1:3; and/or,
the volume of the first reactor is less than or equal to a volume of the material storage tank; and a ratio of the volume of the first reactor to the volume of the material storage tank is optionally 1:(1-5), and is more optionally 1:3; and/or,
the volume of the second reactor is equal to the volume of the material storage tank.

11. The method according to any one of claims 4-10, wherein a frequency of the ultrasonic reactor is from 15 KHz to 60 KHz, and is optionally from 30 KHz to 60 KHz.

12. The method according to any one of claims 4-11, wherein the metal salt required for preparing the ferromanganese oxalate precursor includes a water-soluble ferrous salt, a water-soluble manganous salt, and an optional water-soluble divalent salt of a doping element M, wherein M represents a manganese-doped and iron-doped element, optionally including one or more of Co, Mg, Zn, Ca, Ti, V, Ni, or Cr;
optionally, the water-soluble ferrous salt includes one or more of ferrous chloride, ferrous bromide, ferrous nitrate, ferrous sulfate, ferrous acetate, ferrous fluorosilicate, or ferrous perchlorate;
optionally, the water-soluble manganous salt includes one or more of manganous chloride, manganous bromide, manganous nitrate, manganous sulfate, manganous acetate, or manganous perchlorate; and
optionally, the water-soluble divalent salt of the doping element M includes one or more of a chloride, a nitrate, a sulfate, or an acetate of the doping element M.

13. The method according to any one of claims 4-12, wherein the precipitant includes one or more of oxalic acid or a water-soluble oxalate, and optionally, the water-soluble oxalate includes one or more of lithium oxalate, sodium oxalate, potassium oxalate, and ammonium oxalate.

14. The method according to any one of claims 4-13, wherein,
a concentration of the metal salt solution is from 0.5 mol/L to 2 mol/L, and is optionally from 0.5 mol/L to 1 mol/L; and/or,
a concentration of the precipitant solution is from 0.5 mol/L to 2 mol/L, and is optionally from 0.5 mol/L to 1 mol/L; and/or,
a molar ratio of the metal salt to the precipitant is from 1: 1 to 1:5.

15. The method according to any one of claims 4-14, wherein,
a stirring speed in the first dissolution reactor is from 300 r/min to 600 r/min; and/or,
a stirring speed in the second dissolution reactor is from 300 r/min to 600 r/min; and/or,
a stirring speed in the first reactor is from 300 r/min to 600 r/min; and/or,
a stirring speed in the second reactor is from 300 r/min to 600 r/min; and/or,
a stirring speed in the material storage tank is from 300 r/min to 600 r/min.

16. The method according to any one of claims 4-15, wherein the protective gas includes nitrogen, an inert gas, or a combination thereof.

17. A ferromanganese oxalate precursor prepared through the method according to any one of claims 4-16, having a chemical formula FeₓMn_{y}M_{1-x-y}C₂O₄ •·2H₂O, 0<x<1, optionally 0.2≤x≤0.5 and 0<y<1, optionally 0.5≤y≤0.8 and 0≤1-x-y<1, and optionally 0<1-x-y≤0.05, wherein M represents a manganese-doped and iron-doped element, optionally including one or more of Co, Mg, Zn, Ca, Ti, V, Ni, or Cr, and the ferromanganese oxalate precursor is electroneutral.

18. The ferromanganese oxalate precursor according to claim 17, wherein,
volumetric particle sizes Dv90 and Dv50 of the ferromanganese oxalate precursor satisfy 1<Dv90/Dv50≤2, and optionally 1.3≤Dv90/Dv50≤1.7; and/or,
the volumetric particle size Dv50 of the ferromanganese oxalate precursor is from 200 nm to 600 nm, and is optionally from 230 nm to 510 nm; and/or,
the volumetric particle size Dv90 of the ferromanganese oxalate precursor is from 260 nm to 800 nm, and is optionally from 320 nm to 730 nm.

19. A method for preparing a lithium iron manganese phosphate, at least comprising steps of:
S10: sufficiently mixing the ferromanganese oxalate precursor prepared through the method according to any one of claims 4-16 or the ferromanganese oxalate precursor according to any one of claims 17-18 with a lithium source, a phosphorus source, an optional source of a doping element N, an optional source of a doping element Q, and an optional source of a doping element R at a predetermined ratio to obtain mixed raw materials, wherein N represents a lithium-doped element, optionally including one or more of Zn, Al, Na, K, Mg, Nb, Mo, and W, Q represents a phosphorus-doped element, optionally including one or more of B, S, Si, and N, and R represents an oxygen-doped element, optionally including one or more of S, F, Cl, and Br; and
S20: sintering the mixed raw materials obtained in S10 to obtain the lithium iron manganese phosphate,
wherein the lithium iron manganese phosphate has a chemical formula LiₐN_{b}FeₓMn_{y}M_{1-x-y}P₁₋ₘQₘO₄₋ₙRₙ, M represents a manganese-doped and iron-doped element, optionally including one or more of Co, Mg, Zn, Ca, Ti, V, Ni, or Cr, N represents a lithium-doped element, optionally including one or more of Zn, Al, Na, K, Mg, Nb, Mo, and W, Q represents a phosphorus-doped element, optionally including one or more of B, S, Si, and N, R represents an oxygen-doped element, optionally including one or more of S, F, Cl, and Br, 0.9≤a≤1.1, 0≤b≤0.1, optionally 0<b≤0.05 and 0<x<1, optionally 0.2≤x≤0.5 and 0<y<1, optionally 0.5≤y≤0.8 and 0≤1-x-y<1, optionally 0<1-x-y≤0.05 and 0≤m≤0.1, optionally 0<m≤0.05 and 0≤n≤0.1, and optionally 0<n≤0.05, and the lithium iron manganese phosphate is electroneutral.

20. The method according to claim 19, wherein in S10, a carbon source is also added into the mixed raw materials.

21. A lithium iron manganese phosphate prepared through the method according to claim 19 or 20.

22. A secondary battery, comprising the lithium iron manganese phosphate prepared through the method according to claim 19 or 20.
